# EUROPEAN PATENT SPECIFICATION

(11) **EP 0 364 567 B1**
(45) Date of publication and mention of the grant of the patent: **06.09.1995**
(21) Application number: 89904969.6
(22) Date of filing: 13.04.1989
(51) Int. Cl.: A61B 5/08

(54) **METHOD AND APPARATUS FOR INHALATION OF TREATING GAS AND SAMPLING OF EXHALED GAS FOR QUANTITATIVE ANALYSIS**
VERFAHREN UND VORRICHTUNG ZUR INHALATION VON THERAPEUTISCHEN GASEN UND ZUR PROBEENTNAHME AUSGEATMETER GASE ZUM ZWECKE DER QUANTITATIVEN ANALYSE
PROCEDE ET APPAREIL D'INHALATION DE GAZ DE TRAITEMENT ET D'ECHANTILLONNAGE DE GAZ EXHALES POUR EFFECTUER UNE ANALYSE QUANTITATIVE

(30) Priority: 15.04.1988 US 181814
(43) Date of publication of application: 25.04.1990
(73) Proprietor: SALTER LABS, Arvin, California 93203 (US)
(72) Inventor: Bowe, Edwin A., Columbia, SC 29212 (US); Klein, E.F. Jr., Little Rock, AR 72227 (US); Boysen, Philip G., Chapel Hill, NC 27516 (US)
(74) Representative: Cundy, Anthony Brian
(86) International application number: US8901555
(87) International publication number: WO8909565

(56) References cited:
- WO-A-84/01293
- US-A- 2 750 938
- US-A- 3 513 844
- US-A- 4 440 177
- US-A- 4 602 644
- Anesthesiology, Volume 63, No. 5, issued November 1985, E. IBARRA and D. LEES, "Mass Spectrometer Monitoring of Patients with Regional Anesthesia," see pages 572-573.

## Description

### TECHNICAL FIELD

The present invention relates to medical treatment and monitoring of a living body, and more particularly to an apparatus and method for insufflating a treating gas into the inhalation of a living body and sampling the exhalation of the living body for quantitative analysis of at least one gaseous component thereof.

### BACKGROUND OF THE INVENTION

When a living body, such as a human patient, is sick or being operated upon, it is often necessary to supplement the body's inhalation with a treating gas, such as oxygen or a gaseous anesthetic. In these instances, an accurate quantitative determination of the amount of at least one gaseous component, such as carbon dioxide, in the blood passing through the pulmonary alveoli of the living body is highly desirable. In intensive care situations or under a regional or general anesthetic, an accurate determination of the composition of the breathing gas in the pulmonary alveoli allows bodily functions of a patient to be more readily supervised and treatment of the patient more favorably adapted to the state of those functions. Accurate measurements of at least one gaseous component in the exhalation of a living body also may help improve related diagnostic methods for determining bodily conditions. Measuring the concentration of at least one gaseous component in exhaled breathing gas may be conducted continuously to provide relatively short response times and to enable rapid alterations in an ongoing medical procedure, thereby preventing adverse effects or damage to the living body.

One area of particular interest is the monitoring of end-tidal carbon dioxide, which is the partial pressure of the carbon dioxide component of exhaled gas at the end of exhalation in a spontaneously breathing patient. The quantitative monitoring of end-tidal carbon dioxide in spontaneously breathing patients who are unintubated (those not requiring intubation with an endotracheal tube) would be particularly useful for those unintubated patients who while awake are being treated with supplemental oxygen administration and are receiving regional or local anesthesia or are in a recovery room during emergence from residual general anesthesia. However, previously proposed devices for combined sampling and oxygen administration, while allowing general qualitative detection of carbon dioxide in exhaled breathing gas (and therefore a determination of apnea), have not allowed a quantitative analysis of the carbon dioxide which correlates adequately with the actual amount of this gaseous component in the arterial blood. Consequently, it has not been possible heretofore by breath sampling from an awake patient receiving supplemental oxygen to quantitatively determine the magnitude of respiratory depression occurring as a result of local or regional anesthesia or intravenous sedation.

Prior techniques for insufflating a treating gas into the breathing gas of a patient and simultaneously measuring at least one gaseous component of the exhalation of the patient have involved withdrawing a breathing gas sample through a chamber or conduit receiving both exhaled gas and at least some amount of the insufflated treating gas. For example, the breathing gas sample has been withdrawn from an oxygen mask over the patient's nose and mouth as illustrated by the article of Huntington, et al., in Anesthesiology 65:565-566, 1986. Huntington, et al., inserted an ordinary IV catheter through one of the side ports of a disposable oxygen mask to a point close to the patient's nose and connected it to the sampling tube of a mass spectrometer. According to the authors, the technique was "as satisfactory, but simpler" in comparison with both the Iberra, et al., and the Norman, et al., devices described below.

In an article by Iberra and Lees in Anesthesiology 63:572-573, 1985, there is described a device wherein the sampling catheter of a mass spectrometer is inserted into one prong of the pair of prongs of a conventional nasal cannulae. Although the authors suggest that a sampling catheter so arranged may be used to monitor ventilatory exchange during regional anesthesia, our attempts to use this arrangement for quantitative measurements were unsuccessful because of excessive differences between measured values of end-tidal carbon dioxide and measured values of arterial carbon dioxide.

It had been previously recognized that the differences between arterial carbon dioxide values and end-tidal carbon dioxide values as measured with the Iberra and Lees arrangement were too excessive and erratic to provide a quantitative indication of arterial carbon dioxide. This problem led other researchers in the field, such as Huntington, et al., supra, to conclude that the Iberra and Lees arrangement was unsatisfactory and to try other approaches to achieving a device for quantitative measurements of end-tidal carbon dioxide in unintubated patients while administering supplemental oxygen. Also, in a subsequent article in Anesthesiology 64:664, 1986, Norman, et al., suggest as an alternative to the "unsatisfactory" Iberra and Lees arrangement, that the tip of a sampling catheter (with the proximal connector removed as in Iberra and Lees) be sutured 1 cm. from the pharyngeal opening of a conventional nasal airway. A nasal airway is highly uncomfortable because it completely fills and blocks a nasal passage of the patient. Therefore, as is noted in this article, insertion of the airway requires a "lubricant containing local anesthetic". Although the modified nasal airway device produced a "satisfactory ET CO₂ curve" more consistently than the Iberra and Lees arrangement, "neither method is as reliable as monitoring ET CO₂ via an endotracheal tube". In addition, there is no provision in the Norman, et al., device for insufflating a treating gas such as oxygen.

There is therefore a real need in the art for an insufflating and sampling apparatus having the combined advantageous of insufflating a treating gas into an awake patient and sampling a portion of the patient's exhaled breathing gas in a manner providing a quantitative correlation between measured levels of a gaseous component in the breathing gas and measured levels of the same component in the patient's arterial blood. Neither the Iberra, et al., device apparently nor the Huntington, et al., device fulfill this need because the differences between measured values of end-tidal carbon dioxide in breath samples from these devices and measured values of arterial carbon dioxide are too excessive and erratic to provide a quantitative correlation. The Norman, et al., device apparently also has this deficiency and, in addition, has no provision for insufflating a treating gas simultaneously with sampling of exhaled gas. It is therefore a purpose of the present invention to fulfill the foregoing need.

### SUMMARY OF THE INVENTION

The present invention overcomes the foregoing deficiencies of the prior art and provides a combined adminstration and sampling device for treating gas insufflation into an awake patient and simultaneous sampling giving accurate and reliable quantitative measurements of a gaseous component in exhaled breathing gas. Thus, the nasal cannulae of the present invention is capable of administering oxygen to an awake patient while providing measurements of end-tidal carbon dioxide which are quantitatively equivalent to measurements of end-tidal carbon dioxide obtained by sampling via an endotracheal tube inserted into the trachea (intubation) of a sedated or anesthetized patient. The quantitative measurements available with the present invention thus have a direct correspondence to the actual levels of arterial carbon dioxide. The sampling and analysis regimen may be carried out alone or simultaneously with a regimen for insufflating a treating gas, such as oxygen, into the natural air being inhaled by the patient.

It is believed that the spurious and unreliable results experienced in prior art attempts at such quantitative measurements may be due at least in part to contamination of the exhaled gas sample with the treating gas. Therefore, an object of the present invention is to prevent substantially any leakage or backflow of treating gas into the sampled gas flowpath. This and other objects and advantages are achieved by the method and apparatus of the invention wherein an elongated hollow body, such as a conventional cannulae, is divided into separate inhalation and exhalation manifolds by a transverse wall member within the hollow body, the wall member providing a gas-tight seal positively preventing fluid communication between said inhalation and exhalation manifolds. This apparatus, which thereby has positive sealing means between the flowpath of insufflated treating gas and the flowpath for sampling exhaled breathing gas, provides quantitative measurements of a gaseous component of the exhaled gas which differ only by relatively small and medically insignificant amounts from direct measurements of the gaseous component in corresponding arterial blood samples. The invention thus provides an accurate, consistent and therefore reliable indication of the actual amounts of one or more gaseous component in the arterial blood of a patient.

The invention preferably is in the form of a nasal cannulae having an internal septum located midway between a pair of nasal prongs and extending transversely to sealingly divide the interior of the hollow main body into two entirely separate chambers, one serving as a treating gas manifold in fluid communication with a corresponding nasal prong and the other serving as a sampling gas manifold in fluid communication with the other nasal prong. Although the internal septum is preferably integrally molded with the wall of the main body, the necessary sealing engagement between the septum and the main body may be provided by some other form of adhesion, such as an adhesive composition or a solvent or sonic weld. In all of these alternatives, the sealing means is such that the entire periphery of the septum sealingly engages an abutting wall of the main body to form a continuous gas-tight seal isolating the treating gas manifold from the sampling gas manifold.

The apparatus of the invention further contemplates a first hollow prong in fluid communication with the inhalation manifold, and a second hollow prong in fluid communication with the exhalation manifold. The first hollow prong is adapted to be received in a first nasal passage of a nose of a living body, such as a human body, for insufflating a treating gas, such as oxygen, into the inhaled breath of the living body. The second hollow prong is adapted to be received in a second nasal passage of the nose for withdrawing a portion of the exhaled breath of the living body as a gas sample to be quantitatively analysed. A gas sample conveying means provides substantially all of the withdrawn gas sample to a means for quantitatively measuring the amount of at least one gaseous component, such as carbon dioxide, of the gas sample. This withdrawn portion is sufficiently isolated from the insufflated treating gas for the amount of the gaseous component in the withdawn portion to be substantially directly proportional to the amount of the same gaseous component in the arterial blood of the living body.

The elongated hollow body of the cannulae preferably has a tubular portion provided with an elongated substantially flat base which extends along one side of the tubular portion for supporting the cannulae on a skin surface adjacent to the nose when each of the prongs is positioned within a corresponding nasal passage. The position of the substantially flat elongated base relative to the position and direction of the prongs is such that the support provided by this base tends to keep the cannulae in position relative to the nose. In this embodiment, the hollow tubular portion, and the base portion are preferably of substantially uniform cross-section.

The outer diameter of the base of each of the nasal prongs is substantially smaller than the inner diameter of the naris opening of the corresponding nasal passage such that the prongs do not substantially occlude the inhalation and exhalation of the living body through its nose. This may be achieved by providing nasal prongs of substantial the same shape and size wherein the cross-section of each of the prongs at its base is preferably not more than about one-half, more preferably not more than about one-third, of the cross-sectional area of the naris opening of the corresponding nasal passage in which the nasal prong is to be received.

There are several advantages to providing the cannulae as a new product wherein its hollow body comprises an elongated tube extending substantially perpendicular to the axes of the nasal prongs, and the dividing wall member comprises a septum located intermediate between the opposite ends of this main tube and extending transversely to its wall so as to divide the main tube chamber into inhalation and exhalation manifolds of approximately equal volume. One advantage is that each of the nasal prongs may extend first laterally and then curve approximately in parallel so as to fit comfortably and well within the nasal passages. Another advantage of this embodiment is that the segments of flexible tubing for connecting the inhalation manifold to a source of treating gas and the exhalation manifold to a gas analyzer may be arranged so that neither of these tubing segments passes over either the eyes or the mouth of the living body when the cannulae is properly positioned with the nasal prongs in their corresponding nasal passages.

The new product embodiment also may include a modification in which the sampling nasal prong is extended in length so as to reach a depth in its corresponding nasal passage greater than the depth reached by the other nasal prong to be used for insufflating treating gas. The extended sampling prong may reduce the likelihood that atmospheric air in the region of the naris opening will be sucked into the mouth of the prong during end-tidal sampling of exhaled breathing gas. An influx of atmospheric air into the mouth of the prong and thereby into the sampling manifold at this time may significantly reduce the accuracy of end-tidal measurements.

The invention is particularly useful for insufflating oxygen as the treating gas and for measuring carbon dioxide as the gaseous component of exhalation. When used in this fashion, the inhalation manifold is connected by a segment of flexible tubing to a source of oxygen gas, and the sampling or exhalation manifold is connected by another segment of flexible tubing to a gas analyzer, such as an infrared capnometer or a mass spectrometer, for quantitatively measuring the amount of carbon dioxide in the withdrawn portion of the exhaled breathing gas. Preferably, oxygen is fed continuously into one nasal passage via the inhalation manifold and a portion of the breathing gas is withdrawn continuously from the other nasal passage via the exhalation manifold, the peak partial pressure of measured carbon dioxide generally being considered as the end-tidal value.

### BRIEF DESCRIPTION OF THE DRAWINGS

The invention may be better understood with reference to the detailed description of the preferred and other embodiments taken in conjunction with the accompanying drawings in which:
Fig. 1 is a top perspective view of a nasal cannulae according to the invention;
Fig. 2 is a front elevational view in partial section of a modification to the nasal cannulae of Fig. 1;
Fig. 3 is a top perspective view of the cannulae of Fig. 1 or Fig. 2 illustrating use of the cannulae with a patient and its connection to gas supply and exhalation monitoring apparatuses;

### DESCRIPTION OF A PREFERRED AND OTHER EMBODIMENTS

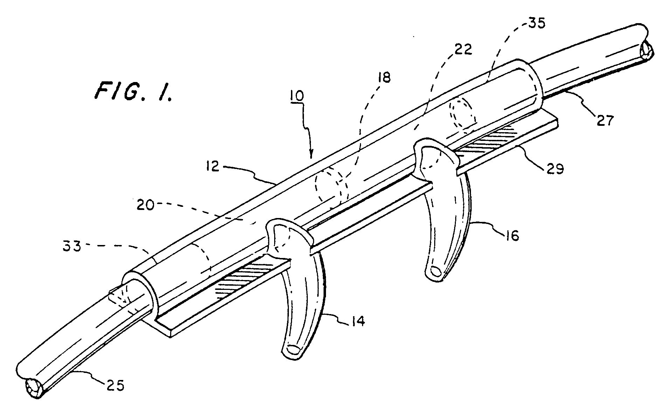

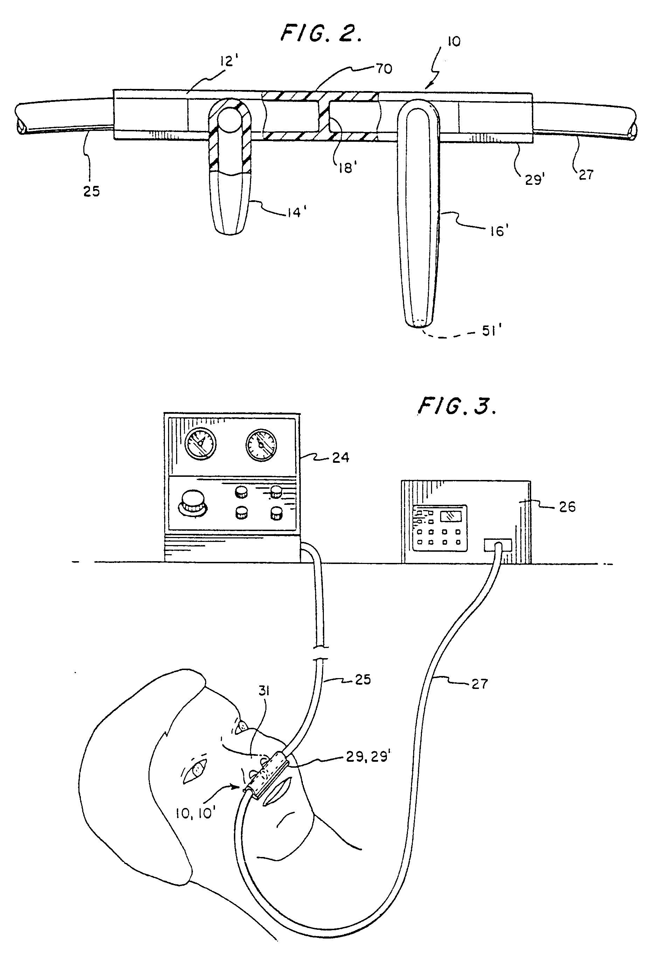

Referring to Fig. 1, there is shown a nasal cannulae, generally designated 10, having a hollow body 12 and a pair of hollow nasal prongs 14 and 16, each adapted to fit within a corresponding nasal passage of the nose of a human being as illusrated in Fig. 3. Sealingly engaging the inner surface of the interior chamber of hollow body 12 is a solid septum 18 which serves as an intermediate transverse wall dividing the interior chamber into an inhalation manifold 20 and an exhalation manifold 22.

The septum 18 is made of a substantially impermeable material and prevents fluid communication between the exhalation and inhalation manifolds. The entire cannulae 10 is preferably molded from a flexible plastic material, septum 18 being integrally molded with the wall of hollow body 12 as seen best in Fig. 2. However, septum 18 may be a separate wall member sealingly adhered to the wall of hollow body 12 by other means, such as by an adhesive composition or by fusion of the material of the septum to the material of the hollow body by solvent welding, sonic welding or the like.

Referring again to Figs. 1 and 3, inhalation manifold 20 may be connected to an oxygen flow regulating device 24 by a segment of flexible tubing 25, and exhalation sampling manifold 22 may be connected to a breathing gas analyzer 26 via another segment of flexible tubing 27. Flexible tubing segments 25 and 27 may be obtained separately or may be supplied as part of the cannulae, in which case end portion 33 of segment 25 and the end portion 35 of segment 27 may be permanently fixed by adhesion to the corresponding end portions of cannulae body 12. The tubing ends 33 and 35 may be sealingly fixed in place by means similar to those for sealingly securing septum 18 in place within hollow body 12. On the other hand, if cannulae 10 is to be supplied without attached tubing, body 12 preferably is made of a material that is more elastic than the tubing ends 33 and 35 such that the force required for seating the ends of separately supplied tubing segments will provide airtight sealing engagement between the respective ends and the wall of the corresponding manifold.

Cannulae 10 also may include an elongated, substantially flat base member 29 for supporting hollow body 12 on the upper lip of a patient with nasal prongs 14 and 16 positioned within the corresponding nasal passages of the patient's nose 31. The position of base member 29 relative to the position and direction of the prongs 14 and 16 is such that the support provided by base member 29 tends to keep the cannulae and its component parts in their correct position relative to nose 31 and the nasal passages therein.

In the embodiment of Fig. 1, the tubing end 33 is aligned with and received within a corresponding portion of inhalation manifold 20. Similarly, the tubing end 35 is aligned with and received within a corresponding portion of exhalation manifold 22. As seen best in the illustration of Fig. 3, this alignment of the discharge end of the oxygen supply tubing and the inlet end of the sampling tubing with the longitudinal axis of tubular body 12 has the advantage of routing both lengths of tubing so that they do not pass over either they eyes or mouth of the patient.

In Fig. 2, there is shown in partial section a modification of the cannulae of Fig. 1 wherein the length of sampling prong 16' has been extended relative to the length of prong 14' for administering treating gas. The amount of the extension may be any amount up to about 100%, preferably about 20 to 60%, more preferably about 50%, relative to the standard length of prong 14'. The mouth 51' of extended prong 16' is positioned to withdraw gas samples from a greater depth within the corresponding nasal passage when modified cannulae 10' is in the same position of use as cannulae 10 in Fig. 3. Withdrawing the exhalation gas sampling from a depth within the nasal passage greater than that reached by conventional nasal prongs may help to ensure that this gas sample has not been diluted with atmospheric air which is always present in and around the opening of the naris. An extension greater than 100% may result in contamination of exhaled gas amples with insufflated treating gas from the central skull chamber which normally receives air from both nostrils. Fig. 2 also shown a septum 18' integrally molded with the wall 70 of tubular body 12' of cannulae 10'.

The availability of the present invention in combination with infrared capnography or mass spectrometry to obtain reliable measurement of a component of exhaled gases during supplemental oxygen administration thus provides a new methodology for non-invasive monitoring of the respiratory status of living bodies, particularly human beings and warmblooded animals. The present invention is believed to afford better patient management for those individuals recovering from general anesthesia and those undergoing a variety of procedures as awake patients under local or regional anesthesia. The present invention is particularly advantageous for use during relatively short-term regional procedures, such as cataract surgery. It is also believed that the invention is appropriate for use in longer term settings, such as for monitoring awake patients in an intensive care unit.

In using the invention, it is important to be cognizant of factors which may affect the accuracy of monitoring end-tidal carbon dioxide. For example, accurate monitoring of end-tidal carbon dioxide in one nostril while insufflating oxygen into the other nostril requires the presence of an intact nasal septum. This is because the present invention relies on the nasal septum to isolate the insufflated oxygen from the exhaled gases. It is believed that a perforated nasal septum may allow sufficient mixing of insufflated oxygen with exhaled gases to give spuriously low end-tidal carbon dioxide measurements, which in turn would produce an unacceptably large arterial to end-tidal difference. In addition, obstruction of the nose or sampling line with bodily secretions may lead to spuriously low measurements. Another source of potential error is the occurrence of predominantly mouth breathing as previously discussed. Certain pathological conditions,such as pulmonary embolism, also may increase the differences between end-tidal and arterial measurements, regardles of the sampling device used.

It also should be recognised that administration of a given flow of oxygen through one nostril instead of two may have a greater drying effect on the single nostil to which oxygen is delivered. Thus, the amount of oxygen which can be successfully delivered for a long period of time through one nostril may be substantially less than that which could be delivered through two nostrils. For example, the administration of 3 litres per minute of oxygen through one nostril will have a drying effect on that nostril equivalent to the administration of 6 litres per minute through both nostrils. Thus, the quantity of oxygen that can be delivered to the patient is limited to the amount that can be fed through one nostril without undue crusting of nasal secretions and/or undue drying of nasal mucosa.

## Claims

1. An apparatus for insufflating a treating gas into the nose of a patient and measuring carbon dioxide in the exhalation of the patient, said apparatus characterised by:
an elongated hollow body (12; 12') including a tubular portion adapted to be received on the skin surface adjacent the nose (31);
a transverse wall (18; 18') within said hollow body (12; 12') defining therein axially separated inhalation and exhalation manifolds (20,22), said wall (18;18') providing a gas-tight seal positively preventing fluid communication between said inhalation and exhalation manifolds (20,22);
supply means (24), connected to said inhalation manifold (20);
a first hollow prong (14;14') in fluid communication with said inhalation manifold (20) and adapted to be received in a first nasal passage of the nose (31) for insufflating said treating gas into the nose (31);
a second hollow prong (16;16') in fluid communication with said exhalation manifold (22) and adapted to be received in a second nasal passage of the nose (31) for withdrawing a portion of the exhalation therefrom, said prongs (14,16;14',16') each being substantially smaller in diameter than the respective nasal passages, so as not to occlude said passages; and
means (27), connected to the exhalation manifold (22) at the end of the hollow body (12; 12') remote from that connected to the supply means (24), for delivering substantially all of said withdrawn portion to a means (26) for quantitatively measuring the amount of carbon dioxide of said withdrawn portion, the amount of said carbon dioxide in said withdrawn portion being substantially directly proportional to the amount of carbon dioxide in the arterial blood of said patient.

2. An apparatus according to Claim 1 characterised in that the outside diameters of said nasal prongs (14,16;14',16') are substantially equal and each of said prongs (14,16;14',16') is sized to have a cross-sectional area which is less than about one-half of the cross-sectional area of the corresponding nasal passage in which said nasal prong (14,16;14',16') is adapted to be received.

3. Method of monitoring end tidal CO₂ in unintubated, conscious, spontaneously breathing patients who are receiving supplemental oxygen by means of a nasal cannula (10) during the administration of local or regional anesthesia or during recovery from residual general anesthesia characterised by the steps of:
providing a nasal cannula (10) on a patient, said cannula (10) having an elongated hollow body (12;12'); a transverse wall (18;18') within said hollow body (12;12') defining therein axially separated inhalation and exhalation manifolds (20,22), said wall (18;18') providing a gas tight-seal positively preventing fluid communication between said inhalation and exhalation manifolds (20,22); supply means (24) including first conduit means (25) communicating with said inhalation manifold (20) and a source of oxygen, insufflation means, including second conduit means (27,27'), communicating with said exhalation manifold (22) and with a means (26) for detecting and measuring the partial pressure of carbon dioxide, said elongated hollow body (12;12') in addition containing separate hollow nasal prong means (14,16;14',16') each communicating with one of said inhalation or exhalation manifolds (20,22) and with respectively each nostril of the patient;
insufflating carbon dioxide from said patient through one of said nasal prong means (16;16') into said exhalation manifold (22) of said elongated hollow body (12;12'), through said second conduit means (27; 27') and into said means (26) for detecting and measuring the partial pressure of carbon dioxide;
whereby the partial pressure of carbon dioxide at the end of the patient's exhalation is measured thereby providing a clinical approximation of the partial pressure of arterial carbon dioxide (PaCO₂).

## Patentansprüche

1. Apparat zum Einblasen eines Behandlungsgases in die Nase eines Patienten und Messen des Kohlendioxids in der ausgeatmeten Atemluft des Patienten, gekennzeichnet durch
einen länglichen Hohlkörper (12; 12') mit einem rohrförmigen Abschnitt zur Anlage an der Hautoberfläche neben der Nase (31); eine querverlaufende Wand (18; 18') innerhalb des Hohlkörpers (12; 12'), die darin axial voneinander getrennte Einatmungs- und Ausatmungs-Verteilrohre (20, 22) begrenzt, wobei die Wand (18, 18') eine gasdichte Dichtung schafft, die eine Fluidverbindung zwischen dem Einatmungs- und Ausatmungs-verteilrohr (20, 22) gezielt verhindert;
eine Zufuhreinrichtung (24), die mit dem Einatmungs-Verteilrohr (20) verbunden ist;
eine erste hohle Zinke (14; 14'), die in Fluidverbindung mit dem Einatmungs-Verteilrohr (20) steht und dafür bestimmt ist, in einen ersten Nasengang der Nase (31) zum Einblasen des Behandlungsgases in die Nase (31) eingeführt zu werden;
eine zweite hohle Zinke (16; 16'), die in Fluidverbindung mit dem Ausatmungs-Verteilrohr (22) steht und dafür bestimmt ist, in einen zweiten Nasengang der Nase (31) zum Abziehen eines Teiles der ausgeatmeten Atemluft eingeführt zu werden, wobei jede der Zinken (14, 16; 14', 16') im Durchmesser wesentlich kleiner als der jeweilige Nasengang ist, um die Gänge nicht zu verschließen; und
eine Einrichtung (27), die mit dem Ausatmungs-Verteilrohr (22) an dem Ende des Hohlkörpers (12; 12') verbunden ist, das von demjenigen Ende entfernt ist, das mit der Zufuhreinrichtung (24) verbunden ist, um praktisch alles von dem abgezogenen Teil einer Einrichtung (26) zum quantitativen Messen der Kohlendioxidmenge des abgezogenen Teils zuzuführen, wobei die Kohlendioxidmenge in dem abgezogenen Teil weitgehend direkt proportional zu der Kohlendioxidmenge im arteriellen Blut des Patienten ist.

2. Apparat nach Anspruch 1, dadurch gekennzeichnet, daß die Außendurchmesser der Nasenzinken (14, 16; 14', 16') praktisch gleich sind, und jede Zinke (14, 16; 14', 16') so bemessen ist, daß sie eine Querschnittsfläche hat, die kleiner als ungefähr die Hälfte der Querschnittsfläche des entsprechenden Nasengangs ist, in welchen die Nasenzinke (14, 16; 14', 16') einführbar ist.

3. Verfahren zum Überwachen des endtidalen CO₂ bei nicht intubierten, bei Bewußtsein sich befindlichen, spontan atmenden Patienten, die zusätzlichen Sauerstoff mittels einer Nasenkanüle (10) während der Verabreichung einer lokalen oder regionalen Narkose oder während des Erwachens aus dem Rest einer Vollnarkose erhalten, gekennzeichnet durch die Schritte:
Bereitstellen einer Nasenkanüle (10) an einem Patienten, wobei die Kanüle (10) einen länglichen Hohlkörper (12; 12'); eine querverlaufende Wand (18; 18') innerhalb des Hohlkörpers (12; 12'), die darin axial voneinander getrennte Einatmungs- und Ausatmungs-Verteilrohre (20, 22) begrenzt, wobei die Wand (18; 18') eine gasdichte Dichtung schafft, die eine Fluidverbindung zwischen dem Einatmungs- und Ausatmungs-Verteilrohr (20, 22) gezielt verhindert; und eine Zufuhreinrichtung (24) hat, die eine erste Leitung (25), die mit dem Einatmungs-Verteilrohr (20) und einer Sauerstoffquelle in Verbindung steht, und eine Einblaseinrichtung aufweist, die eine zweite Leitung (27, 27') einschließt, die mit dem Ausatmungs-Verteilrohr (22) und einer Einrichtung (26) zum Feststellen und Messen des Teildrucks des Kohlendioxids in Verbindung steht, wobei der längliche Hohlkörper (12; 12') zusätzlich getrennte hohle Nasenzinken (14, 16; 14', 16') enthält, von denen jede mit dem Einatmungs-oder Ausatmungs-Verteilrohr (20, 22) und mit dem jeweiligen Nasenloch des Patienten in Verbindung steht;
Einblasen von Kohlendioxid von dem Patienten durch eine der Nasenzinken (16; 16') in das Ausatmungs-Verteilrohr (22) des länglichen Hohlkörpers (12; 12') durch die zweite Leitung (27; 27') und in die Einrichtung (26) zum Feststellen und Messen des Teildrucks des Kohlendioxids;
wodurch der Teildruck des Kohlendioxids am Ende der Ausatmung des Patienten gemessen wird, um dadurch eine klinische Annäherung des Teildrucks von arteriellem Kohlendioxid (PaCO₂) zu erhalten.

## Revendications

1. Appareil pour insuffler un gaz traitant dans le nez d'un patient et mesurer le dioxyde de carbone dans l'exhalation du patient, ledit appareil étant caractérisé par :
un corps creux allongé (12, 12') comportant une portion tubulaire adaptée pour être reçue sur la surface de peau adjacente du nez (31);
une paroi transversale (18, 18') à l'intérieur dudit corps creux (12, 12') définissant à l'intérieur de celui-ci des collecteurs (20, 22) axialement séparés d'inhalation et d'exhalation, ladite paroi (18, 18') fournissant un joint étanche au gaz évitant de manière positive une communication de fluide entre lesdits collecteurs (20, 22) d'inhalation et d'exhalation ;
des moyens d'alimentation (24) reliés audit collecteur (20) d'inhalation ;
une première dent de fourche (14, 14') creuse en communication de fluide avec ledit collecteur (20) d'inhalation et adaptée pour être reçue dans un premier passage nasal du nez (31) pour insuffler ledit gaz traitant dans le nez (31);
une seconde dent de fourche (16, 16') creuse en communication de fluide avec ledit collecteur (22) d'exhalation et adaptée pour être reçue dans un second passage nasal du nez (31) pour retirer une partie de l'exhalation de celui-ci, lesdites dents de fourche (14, 16 ; 14', 16') étant chacune substantiellement plus petites en diamètre que les passages nasaux respectifs, de manière à ne pas obstruer lesdits passages ; et
des moyens (27) reliés au collecteur (22) d'exhalation à l'extrémité du corps creux (12 ; 12') éloignés de ceux reliés aux moyens (24) d'alimentation pour délivrer sensiblement toute ladite portion retirée vers des moyens (26) pour mesurer quantitativement la quantité de dioxyde de carbone de ladite portion retirée, la quantité dudit dioxyde de carbone dans ladite portion retirée étant substantiellement directement proportionnelle à la quantité de dioxyde de carbone dans le sang artériel dudit patient.

2. Appareil selon la revendication 1, caractérisé en ce que les diamètres extérieurs des dents de fourche nasales (14, 16 ; 14', 16') sont substantiellement égales et chacune desdites dents de fourche (14, 16; 14', 16') est dimensionnée pour avoir une aire en section transversale qui est inférieure à environ la moitié de l'aire en section transversale du passage nasal correspondant dans lequel ladite dent de fourche nasale (14, 16 ; 14', 16') est adaptée pour être reçue.

3. Procédé de contrôle de dioxyde de carbone de fin de respiration dans des patients respirant spontanément conscients et non équipés de tubes internes qui reçoivent de l'oxygène supplémentaire au moyen d'une canule nasale (10) pendant l'administration d'un anesthésiant local ou régional ou pendant la récupération d'un anesthésiant général résiduel, caractérisé par les étapes de :
fournir une canule nasale (10) à un patient, ladite canule (10) ayant un corps allongé creux (12 ; 12'), une paroi transversale (18 ; 18') à l'intérieur dudit corps creux (12 ; 12') définissant dans celui-ci des collecteurs (20, 22) séparés axialement d'inhalation et d'exhalation, ladite paroi (18 ; 18') fournissant un joint étanche au gaz empêchant de manière positive une communication de fluide entre lesdits collecteurs (20, 22) d'inhalation et d'exhalation, des moyens d'alimentation (24) comportant des premiers moyens de conduit (25) communiquant avec ledit collecteur d'inhalation (20) et une source d'oxygène, des moyens d'insufflation comportant des seconds moyens de conduit (27, 27') communiquant avec ledit collecteur (22) d'exhalation et avec des moyens (26) pour détecter et mesurer la pression partielle du dioxyde de carbone, ledit corps creux allongé (12 ; 12') contenant en plus des moyens de dents de fourche nasaux creux séparés (14, 16 ; 14', 16') chacun communiquant avec l'un des collecteurs (20, 22) d'inhalation ou d'exhalation et avec chaque narine du patient ;
insuffler du dioxyde de carbone depuis ledit patient à travers l'un desdits moyens de dents de fourche nasaux (16 ; 16') dans ledit collecteur d'exhalation (22) dudit corps creux allongé (12 ; 12') à travers lesdits seconds moyens de conduit (27 ; 27') et dans lesdits moyens (26) pour détecter et mesurer la pression partielle de dioxyde de carbone ;
de sorte que la pression partielle du dioxyde de carbone à la fin de l'exhalation du patient est mesurée fournissant ainsi une approximation clinique de la pression partielle du dioxyde de carbone artériel (PaCO₂).
